# EUROPEAN PATENT SPECIFICATION

(11) **EP 4 150 351 B1**
(45) Date of publication and mention of the grant of the patent: **15.04.2026**
(21) Application number: 21803836.2
(22) Date of filing: 10.05.2021
(51) Int. Cl.: G01N 33/94, B01L 3/02, G01N 33/50, G01N 30/88, G01N 30/72, G01N 30/90, G01N 33/487

(54) **METHOD OF TESTING TEARS AS A BIOMARKER FOR CANNABINOIDS**
VERFAHREN ZUM TESTEN VON TRÄNEN ALS BIOMARKER FÜR CANNABINOIDEN
PROCÉDÉ DE TEST DE LARMES EN TANT QUE BIOMARQUEUR DE CANNABINOÏDES

(30) Priority: 11.05.2020 US 202063022874 P
(43) Date of publication of application: 22.03.2023
(73) Proprietor: Valenti, Denise A., Quincy, MA 02169 (US)
(72) Inventor: Valenti, Denise A., Quincy, MA 02169 (US)
(74) Representative: Patentanwälte Geyer, Fehners & Partner mbB
(86) International application number: PCT/US2021/031613
(87) International publication number: WO 2021/231318

(56) References cited:
- US-A1- 2005 232 813
- US-A1- 2006 188 410
- US-A1- 2015 346 170
- US-A1- 2016 282 371
- US-A1- 2019 021 704
- YETISEN ALI K. ET AL: "Paper-based microfluidic system for tear electrolyte analysis", LAB ON A CHIP, vol. 17, no. 6, 16 February 2017 (2017-02-16), UK, pages 1137 - 1148, XP093147958, ISSN: 1473-0197, DOI: 10.1039/C6LC01450J
- YETISEN ALI K. ET AL: "Paper-based microfluidic system for tear electrolyte analysis: supporting information", LAB ON A CHIP, vol. 17, no. 6, 16 February 2017 (2017-02-16), UK, pages 1 - 17, XP093147943, ISSN: 1473-0197, DOI: 10.1039/C6LC01450J
- RANTAM�KI ANTTI H. ET AL: "Human Tear Fluid Lipidome: From Composition to Function", PLOS ONE, vol. 6, no. 5, E19553, 5 May 2011 (2011-05-05), US, pages 1 - 7, XP093148151, ISSN: 1932-6203, DOI: 10.1371/journal.pone.0019553
- TAORMINA ET AL: "Analysis of Tear Glucose Concentration with Electrospray Ionization Mass Spectrometry", JOURNAL OF THE AMERICAN SOCIETY FOR MASS SPECTROMETRY, ELSEVIER SCIENCE INC, US, vol. 18, no. 2, 27 January 2007 (2007-01-27), pages 332 - 336, XP005863628, ISSN: 1044-0305, DOI: 10.1016/J.JASMS.2006.10.002

## Description

### TECHNICAL FIELD

Embodiments of the invention relate to testing for presence of cannabinoids from the consumption of cannabis in biologic samples and to testing for impairment arising from the consumption of cannabis and other psychoactive drugs. More particularly, the invention relates to testing for impairment caused by the consumption of such substances to identify individuals who are impaired for driving and other tasks.

### BACKGROUND

There are multiple cannabinoids in a marijuana plant. There are also multiple forms of metabolites within the human system. For ease of discussion this application may discuss three forms of cannabinoids: anandamide, which is a natural endogenous cannabinoid in humans, Δ9-tetrahydrocannabinol (Delta-THC), which is the main psychoactive component of marijuana, and 11-nor-9-carboxy-THC (THC-COOH), which is an inactive metabolite. THC is not an easy drug to measure. The presence of THC-COOH within the human body is only used to prove that a person has used marijuana in the past. The most common biologic sample that is tested for the presence of THC is blood. However, if a driver refuses to permit a blood draw to test for the presence of THC it can take up to four hours, and sometimes longer, to get a court order to obtain a blood sample for testing. A May 2016 AAA Foundation study found that it takes an average of 165 minutes from first contact with police until blood is drawn for a test. The study also indicates that participants' THC blood levels dropped, on average, 5 ng/mL during the first two hours after using cannabis.

Legalization of medical and recreational use of marijuana and related compounds has generally tended to cause an increase in driving following consumption. For example, according to a National Public Radio (NPR) report, the Washington State toxicology lab saw a drastic increase in the number of blood samples tested positive for marijuana in the first three years after legalization-from 19 percent to 33 percent. There is an even larger increase in detected components of marijuana when only active THC (i.e., THC molecules which have not yet been in the system long enough to be metabolized) is considered. Before legalization, there was active THC detected in about half of all blood samples tested for marijuana after an accident involving fatalities. In 2015, the Washington Traffic Safety Commission told NPR they found active THC in about 85 percent of all tested samples after a deadly accident.

There are limited options available to detect impairments impacting driving when impairment is due solely to the use of psychoactive drugs, particularly cannabis. Yet cannabis users are ten times more likely to be injured in automobile accidents than non-users. In a 2007 roadside survey, more drivers demonstrated positive test results for drugs than alcohol, which contributes to the risk of accidents when driving. Eleven percent of those presenting in an emergency room after an auto accident were found to have used cannabis in the absence of other drugs or alcohol. Despite the increased risk of fatality after acute cannabis use, a survey of drivers in Canada found that 4.8 percent of drivers admitted to driving within two hours of using cannabis.

With the current legalization of cannabis for recreational use in several states, the rate of automobile injury secondary to cannabis intoxication is likely to increase. Within the United States, Colorado has the most experience with cannabis use and driving. Starting January 1, 2014 Amendment 64 took effect allowing for the legal use of recreational cannabis in the state of Colorado for citizens over the age of twenty-one. Even though the laws in Colorado clearly state that it is illegal to drive while high, twelve percent of the citations issued in 2014 for DUI involved cannabis use. The DUI fatality rate increased by 6 percent in 2014 and the rate of DUI citations increased by 23 percent compared to 2013. Of the total citations issued, over six percent were suspected to involve only cannabis and no other substances. Colorado officials take the position that any amount of cannabis can impair a person for driving, and that DUI citations can be based on observations. Colorado has established that, legally, 5 nanograms of tetrahydrocannabinol (the component in cannabis impacting cognitive function) per milliliter of whole blood impairs driving function. Other states have adopted zero-tolerance legislation, which is supported by research that indicates how little cannabis is required to significantly impair skills required for driving. The Colorado Department of Transportation performed a survey on the attitudes and behaviors of residents several months before recreational cannabis became legal. The survey found that twenty percent of the respondents that had used cannabis in the previous year had driven shortly after consuming cannabis and those that did so within two hours drove, on average, seventeen times during a month. Colorado invested one million dollars on a public education campaign, "Drive High, Get DUI", in an effort to minimize the impact of driving while impaired secondary to cannabis. Even after the campaign, 57% of those reporting to use cannabis drove within two hours of consumption.

Washington State legalized marijuana more recently than Colorado, but nevertheless reports significant problems with increasing numbers of impaired drivers due to cannabis consumption. A report for the Washington Traffic Safety Commission in a survey conducted by the Pacific Institute for Research and Evaluation found that seventy percent of drivers questioned had used cannabis and, of those reporting use, 44% acknowledged they had driven a car less than two hours after using cannabis. Further, 90% of those who drove while using cannabis did not feel that cannabis impaired their ability to drive. In the year 2012 there were 988 driver tests that came back positive for cannabis and this increased to 1, 362 in 2013. From 2009 through 2013, more than 1,000 people died in impaired driving collisions in Washington. Impaired driving is involved in nearly half of all traffic deaths and more than 20% of serious injury collisions. When cannabis is combined with alcohol, the effect of impairment is more deadly than alcohol alone. Alcohol intoxication increases the risk of fatality in an accident by 13 times when compared to a sober driver, while intoxication from the combination of alcohol and cannabis increases the fatality risk by 24 times.

In 2011, the National Institute on Drug Abuse ("NIDA") prepared a White Paper on drugged driving research that discussed marijuana use. The White Paper explained that, "for illegal drugs, Zero Tolerance (ZT) per se laws are those which set that limit at the drug detection cut-off level." It is not necessary to prove driver impairment to convict an offender under a per se law. NIDA further explained that a "per se drugged driving law is one in which a specified level of a drug in the body of a driver is defined as an offense." The specified level is generally based upon the level at which there is evidence that the drug has been shown to affect driver performance, such as the 0.08g/mL limit for alcohol.

There is limited research on cannabis impairment related to driving. Impairment due to cannabis differs significantly than the impairments caused by alcohol. Even small amounts detected in the blood have been shown to impair the cognitive perceptual functions necessary for safe driving. The demonstration of cognitive dysfunction with even small amounts of cannabis is fueling the fire of ZT legislation. Fifteen states have adopted ZT regulations applicable to drug and cannabis use. Although some jurisdictions exclude medical marijuana users, approximately nine states have adopted more absolute laws that apply the zero-tolerance policy to all cannabis users.

A September 2015 report from the Governor's Highway Safety Administration discussed the complexities of evaluating impairment and driving under the influence of cannabis. Extraordinary attention was paid to cannabis, which is considered a threat to public health safety. The report reiterates that, much like driving under the influence of alcohol ("DUI"), driving under the influence of drugs ("DUID"), is illegal in every state. Another similarity with DUI is that law enforcement must consider two requirements for a DUID. First, the driver must exhibit signs of impairment through behavior observed by a law enforcement officer and the signs of impairment must be linked to a drug. The law enforcement officer must have actually observed the driver demonstrating the signs of impairment before obtaining chemical evidence of a drug (typically via a blood test). The officer must be able to link any chemical evidence of drug presence to the signs of impairment that they observed. If the driver refuses a blood test, the officer relies on observations without corroborating chemical evidence.

Cannabis related driving impairment presents a unique challenge to law enforcement as well as to the court systems. This process is relatively longer than the process for alcohol. With alcohol use, the signs are well understood and backed by years of research. The Standardized Field Sobriety Testing ("SFST") is an efficient and accurate method of screening for impairment. Evidence of blood alcohol levels can be obtained by the biomarkers measurable by a breath test. The links between SFST, breath-testing and alcohol impairment are recognized by the judiciary system. Traditional training of law enforcement officers does not always include adequate training to observe impairment to driving that arises from drugs. Obtaining the requisite legal permissions to draw blood from a driver can take several hours, and there are concerns that the measured levels of cannabis in the blood may not accurately indicate the level of impairment to driving. Further drug testing can be expensive with labs having substantial backlogs.

When there is a concern related to impairment to drive, police officers use standardized observation techniques. The most common is the standardized field sobriety test ("SFST"), which can also be used to detect other impairments to driving, including cannabis. A clear understanding of how cannabis has an impact on the ability to drive and limits on how an officer can compel further testing creates barriers to removing drivers impaired by cannabis consumption from the road. Unless there is evidence of impairment to drive similar to the SFST related to alcohol, an officer cannot require a driver to undergo further evaluations.

A part of the SFST is the screening for alcohol related horizontal gaze nystagmus and other eye movement patterns. The horizontal gaze nystagmus related to alcohol occurs when the eyes move horizontally from looking straight ahead to looking to the side. The observation of a driver's eye movements along the horizontal axis while the driver is fixating at a target are subjectively interpreted for the presence of horizontal nystagmus, jerking eye movement, and eye pursuit dysfunctions. If the nystagmus is observed when the eye position is at an angle approximating forty five degrees or less and/or there are losses of fixation during pursuit eye movements, there is a high likelihood that the driver has a blood alcohol level that would impair driving.

Horizontal gaze nystagmus testing has been shown to have an accuracy of over 75% in the detection of blood alcohol content of 0.09% or greater. Furthermore, studies of horizontal gaze nystagmus have shown the test to be sensitive even if blood alcohol levels are lower than legal limits of .08%. Horizontal gaze nystagmus testing, when properly administered, is sensitive in blood alcohol levels of .04% - .08%. The horizontal nystagmus test is not complex to administer, but it does require significant learned skill to interpret a driver's response. This can create issues when presented in court as the officer's administration and interpretations of the test are subjective. When the signs of horizontal gaze nystagmus occur, there is concern that the blood level is greater than 0.04%. The rate of alcohol related auto accidents is staggering. Over 15,000 deaths associated with alcohol related traffic accidents occur annually, and over 40% of auto fatalities each year are related to alcohol consumption. Cannabis also impairs driving but, unlike alcohol, it is difficult to determine blood levels that cause impairment.

Observation of eye movement as an indicator of impairment for cannabis use is inconclusive. Citek and colleagues evaluated 25 participants identified by urinalysis to have cannabis as the only intoxicant and found that there was lack of findings for deficits in eye movements for horizontal gaze nystagmus. Smooth tracking eye movements and saccadic tracking eye movements are reduced with alcohol but not with marijuana or a placebo in the motion study undertaken by Flom and colleagues. Regarding eye movement dysfunction, there are now observations in the field that with heavy chronic marijuana use or use with verily highly concentrated dabbing products there can be abnormal eye tremors and eye movement, particularly smaller microsaccades. The dysfunctions are different than the eye movement abnormalities seen with alcohol consumption. In a study of 20 adults using cannabis alone and cannabis combined with alcohol, researchers found that with cannabis alone the users showed impairment with the field sobriety test of one leg stand, but dysfunctions related to horizontal gaze nystagmus occurred when cannabis was combined with alcohol. One leg standing is uncertain as to its correlation with cannabis consumption. Accordingly, even though affected by alcohol, horizontal gaze nystagmus appears not to be affected by cannabis.

There is potential to test for the presence of cannabinoids in fluids. Currently, commercial products use technology that tests urine, blood, breath and saliva. Sweat is also a viable fluid showing presence of cannabinoids, but there are no tests or products on the market for sweat. The water or fat content of the fluid is a major factor in the detection of cannabinoids in fluids and the retention of cannabinoids in bodily fluids. Cannabinoids are hydrophobic, which bind longer and stronger to lipids. Blood has minimal lipids and, although cannabinoids bind quickly, blood does not retain the chemical bonds very long. Organs and body tissue, on the other hand, have considerable lipids, so that cannabinoids can bind strongly and remain present in these tissues for days, weeks, or months.

In studies related to athlete hydration that evaluate sensitivity and ease of obtaining samples, saliva, sweat and tears were compared to the standards of blood and urine. Saliva was considered the easiest to obtain but the data suffered from extreme variability. Obtaining sweat was considered to be complex and time consuming but offered more reliable data. Tears were considered to be the easier sample to obtain while also providing reliable data. In particular, collecting tear fluid is easy to access and the collection procedure causes very little discomfort.

Early efforts at creating a biologic test that detects cannabinoids mainly consisted of breathalyzers. One of the breathalyzers already available is not capable of roadside reading and is currently not FDA approved. The breathalyzer, called the Sensbues DrugTrap, is a device that collects the biologic sample for analysis in a lab. The breathalyzer comprises of a filter holder, mouthpiece, plastic bag with volume indicators, and seals for both ends. The user blows into the mouthpiece, then seals and mails the bag to the company for analysis. The delayed results severely limit the utility of this breathalyzer in a roadside law enforcement setting.

However, there are many companies working on a roadside breathalyzer capable of providing immediate results. Research related to roadside breathalyzers includes HoundLabs who showed that cannabinoids can be detected in breath for up to three hours after smoking and identified a correlation between identified cannabinoid concentrations and blood concentrations for that initial three-hour period.

University of Pittsburgh researchers have created technology that identifies disease states in breath using carbon nanotubes and have reported their technology also applies to the presence of cannabinoids. The breathalyzer measures the electrical resistance of semiconductor-enriched carbon nanotubes that are 100,000 times smaller than a human hair and are good at conducting electricity. Cannabinoids will bind to the surface and change the electrical acoustics.

Lifeloc Technologies has prioritized research funding to develop a breathalyzer but does not yet have a product on the market. Current claims are the ability to detect delta-9THC down to 5 nanograms per milliliter in fluid and vapor/breath in a laboratory setting. Two companies in Canada are also working on breathalyzers. SannTek Laboratories uses a nanotechnology strategy and Cannabix Technologies is partnering with researchers at the University of Florida and the University of British Columbia to develop two different cannabinoid breathalyzer devices. Giner labs, based in Boston, have been working to develop a cannabinoid breathalyzer solution for close to a decade.

There are already tests on the market for saliva that measure the presence of multiple drugs. One such test is healthcare company Abbott's SoToxa rapid mobile drug screening test. The handheld device, which analyzes a saliva sample in five minutes, is being used in Canada, Spain and US states such as Michigan, Alabama and Oklahoma. The Dräger DrugTest 5000 system, another roadside saliva test on the market, is a means of testing saliva fluid samples for drugs including amphetamines, designer amphetamines, opiates, cocaine and metabolites, benzodiazepines, cannabinoids, and methadone.

While not used in direct practice, sweat has been studied to determine the viability of measuring quantities of cannabinoids in the system. A study of 12 participants using a patch to collect sweat identified the psychoactive form of Delta-THC in all 12, confirmed with blood analysis, and did not identify any inactive form THC-COOH. Simple drug wipes demonstrate presence of Delta-THC and THC-COOH. Sweat deposits from fingerprints identified Delta-THC in another study. Biomarkers in sweat have been used to identify disease.

Another product, though not on the market, is a telephone application to record and analyze eye movement scanning with cannabis consumption. A further product, BreathalEyes records eye movements and nystagmus and calculates the probable blood alcohol content of the person using the application on a smart phone or tablet. MyCanary, yet another product, is a telephone application that allows a driver to test their own theoretical potential to be too impaired to drive. It utilizes simple cognitive and physical tests including balance, memory, reaction and time perception and provides readouts for the driver. There is no research or science background available on this product and it is not intended to be used by law enforcement or the justice system.

Therefore, there is a significant need for accurate and reliable tests that are easy to use, sensitive and specific to cannabis impairment. Ideally, the results of these tests would be available quickly, from wherever the test was administered, using a bodily fluid that is safe and easy to sample.

The eye has been used post-mortem to identify biomarkers of drug use. Inactive THC has been identified in the vitreous when blood levels were very high. The chemical composition of the vitreous body and fluids vary significantly from tear fluids and the vitreous is inaccessible except by surgical intervention. The vitreous tends to only have biomarkers of inactive THC and, relative to other tissues, has the lowest concentration because of the hydrophobic nature of THC and the limited penetration of intra ocular structures. Conversely, bile has the highest concentration of inactive THC. Other researchers failed to identify any cannabinoids in the vitreous, despite identifying cannabinoids in the blood. A pig model demonstrated inactive THC in the vitreous. Being hydrophobic, THC molecules in tears do not readily cross into the eye through ocular tissues, as demonstrated by topical administration of different cannabinoids and THC.

US 2019/0021704 A1 disclose a cartridge for oral fluid analysis and methods of use. The focus is to analyze salvia or urine. Samples are taken by swabs.

Yetisen A. et al., "Paper-based microfluidic system for tear electrolyte analysis", Lab Chip, 2107, 17, 1137-1148, relates to the analysis of the aqueous fraction of tears in the context of the diagnosis of ocular deceases.

### Background on the Tear Film

The tear film covers the anterior structures of the eye and is generally considered to have a multilayered structure. From the corneal surface outward, the tear film includes a mucin layer, an aqueous layer, and a lipid layer. The mucin layer overlies the surface of the cornea and the conjunctiva and serves to provide an interface between the corneal epithelium, the conjunctival epithelium and the further layers of the tear film. Without the mucin layer, the corneal epithelium is generally considered hydrophobic because it contains considerable amounts of lipid. Overlying the mucin layer is the aqueous layer consisting largely of water and a small amount of salt and various other trace chemicals. Overlying the aqueous layer is the lipid layer formed of secretions of the meibomian glands and other lipid secreting glands located in and around the eyelids. The tear film protects the tissues of the corneal epithelium from drying and also fills in or bridges minor gaps or irregularities in the corneal epithelium to provide a smooth refractive surface interface between the front of the eye and the ambient atmosphere. Thus, the anterior most refractive interface between the air and the eye is actually formed by the tear film rather than the cornea itself.

The interface between the tear film and the atmosphere represents the most powerful optical focusing interface in the eye. The tear film interface with the atmosphere accounts for approximately two thirds of the focusing power the eye. This focusing power varies but, on average, is approximately 43 diopters. Because the focusing power at the tear film interface is so large, small changes in the tear film can have a large effect on refraction and clarity of focusing.

When refraction of light occurs in the eye, the anterior surface of the cornea is generally considered to be the most powerful focusing element of the eye. In fact, focusing primarily occurs at the interface between the tear film and the atmosphere. Accordingly, the tear film is extremely important for refractive reasons in the eye. In addition, the tear film moistens, lubricates, and assists in nourishing and carrying away metabolic debris from the cornea.

Generally, the tear film is considered to include a mucin layer nearest to the cornea and an aqueous layer overlying the mucin layer and a lipid layer overlying the aqueous layer. The middle aqueous layer provides moisture to the corneal tissue, carries important nutrients, and serves to remove metabolic waste produced by the cornea.

The mucin that forms the mucin layer, nearest the cornea, is secreted by goblet cells in the conjunctiva. The conjunctiva is the transparent tissue that covers the sclera and the backside of the eyelids. The mucin layer functions to decrease surface tension of the tear film. In addition, the cornea itself is hydrophobic. Without the mucin layer to provide a bridge between the cornea and the aqueous layer, the aqueous layer would bead up and allow dry spot formation on the cornea.

The aqueous layer is secreted primarily by the glands of Wolfring and Krause located in the eyelid margin. The aqueous layer helps provide an optically smooth, transparent surface to the precorneal tear film. The lipid layer is secreted by the meibomian glands, and the glands of Zeiss and Moll. The glands of Zeiss and Moll are also located at the eyelid margin.

Blinking is essential to maintenance of the precorneal tear film. During each blink, the eyelid wipes over the surface of the cornea, smoothing the mucin layer and spreading the overlying aqueous and lipid layers to provide a completely wetted surface. In between blinks, the tear film thins due to evaporation of the aqueous layer. If evaporation is excessive, dry spots may form on the surface of the cornea.

### Background on the Chemistry of Cannabis

The cannabis plant produces a unique class of chemical compounds called cannabinoids. Cannabinoids are a primary contributor to the plants' psychotropic and therapeutic properties whether the plant is ingested, or its vaporized products are inhaled. Cannabinoids are hydrocarbons. Hydrocarbons consist of hydrogen, carbon, and oxygen atoms. To date, laboratory research has identified at least 113 cannabinoids.

The cannabis plant contains an acidic compound called THCA. THCA has minimal pharmacologic and psychotropic effects. However, when the acidic compound THCA is heated, it is converted to a biologically active compound tetrahydrocannabinol ("delta 9-THC") by the chemical process of de-carboxylation. In the process of decarboxylation, a carboxylic acid functional group is severed from the THCA molecule. In general, a similar process applies to all cannabinoids. Decarboxylation converts acidic CBDA to chemically neutral CBD, and converts CBGA to chemically neutral CBG.

Delta 9-THC, commonly referred to as THC (short for tetrahydrocannabinol), is commonly known because of its psychotropic effects. While blood testing exists to determine the presence of cannabinoids in the blood circulation, it has proven to be of limited value for road-side application because there is no generally accepted concentration of cannabinoids in the blood that is considered impairing. Efforts to test saliva for the presence of cannabinoids have not met with general acceptance and success as cannabinoids do not seem to be excreted in saliva to any significant degree. Urine can be tested for cannabinoid metabolites as well. Carboxy THC (11-nor-9-carboxy-δ-9-THC) is a major metabolite in urine, but is not related to source, time of exposure, amount, or impairment. In fact, there is no known point of comparison between impairment and the presence of cannabinoid metabolites in urine. These metabolites must be water-soluble to be excreted in urine and therefore do not represent the psychoactive cannabinoids.

Accordingly, there are still needs for detecting evidence of the use of cannabinoids that can be used in a field-testing screening technique or tool. In particular, there remain needs for devices and methods capable of providing results quickly and of being used by law enforcement personnel in road-side applications with safety for both the driver and the officer as a high priority.

### SUMMARY

The invention is defined by the independent claim.

According to embodiments of the invention, many of the above problems are addressed. As discussed above, decarboxylation converts acidic CBDA to chemically neutral CBD and CBGA to chemically neutral CBG. Because these substances are chemically neutral, that is, not acidic or alkaline, they tend to be more miscible in fats or lipids than in aqueous solutions. In general, a similar process applies to all cannabinoids. For example, when the acidic compound THCA is heated, it is converted to a biologically active compound tetrahydrocannabinol (Delta-THC) by the chemical process of de-carboxylation. In the process of decarboxylation a carboxylic acid functional group is severed from the THC a molecule. It is the inventor's observation that this is likely the reason that cannabinoids are not readily detected in saliva. Saliva is almost entirely aqueous in nature and thus not a good solute in which to dissolve the chemically neutral cannabinoids that are likely to have psychotropic effects.

Tears are a reasonable option for the bodily fluid to be tested. Reports indicate that tears have biomarkers for identifying disease and pregnancy. Tears are already a viable source of biologic material for forensics purposes. The presence of cannabinoids such as Delta-THC in tears is expected because the conjunctiva is dense with cannabinoid receptors and structures associated with the production of tears such as the lacrimal gland, also have cannabinoid receptors. Further, researchers have identified an association between cannabinoid receptors and conjunctival goblet cells. As part of the epithelial lining of many organs and tissues, goblet cells are distributed throughout the body. Goblet cells are expected to attract and hold cannabinoids such as THC because the cells are lipophilic. Spontaneous blink is reduced with cannabis use, which is conducive to holding the tears longer in the eye. Moreover, the natural endogenous cannabinoid, anandamide, has been identified in the lacrimal gland (i.e., the gland that produces most of the fluid bathing the eye) of a porcine model. The viability of testing tears is further underscored by the Drug Recognition Experts report marijuana users having watery eyes and an excess of tears.

Sampling the tear film provides a fast, painless and noninvasive procedure by which a sample capable of being tested for the presence of cannabinoids may be obtained. The tear film is readily accessible and relatively unskilled persons can be trained to safely gather samples of the tear film. Appropriate packaging and handling of sampling materials are likely to permit sampling with minimal risk of infection or injury to the individual being sampled.

However, tears have a significant lipid component which enhances the binding or dissolution of cannabinoids and thus their bioavailability for sampling. It is generally considered that the anterior most layer of the tear film is composed primarily of lipids. Accordingly, it is expected that cannabinoids that are chemically neutral should be readily miscible or soluble in the lipid component of the tear film.

According to example embodiments of the invention, tear samples may be obtained by use of a micropipette or a capillary tube, A micropipette or capillary tube should be packaged in a sterile fashion for single use.

According to an example embodiment of the invention, the presence of cannabinoids in the tear film can be analyzed by application of color testing. In a further example embodiment, samples of the tear film may be analyzed to identify cannabinoids by the application of gas chromatography. Other example embodiments include analyzing the tear film sample by application of high-performance liquid chromatography, high-pressure liquid chromatography, gas chromatography-flame ionization detection ("GC-FID"), thin layer chromatography ("TLC"), spectrometry, or mass spectrometry.

In yet a further example embodiment of the invention the method of identifying cannabinoids in tear film may further comprise separating a lipid fraction of the tear sample from an aqueous component, a mucin component or both the aqueous and mucin component of the tear sample prior to analysis for the presence of cannabinoids.

The method of obtaining and processing a tear sample from an individual includes obtaining a sample of tears from an individual by use of a flexible tapered polymer structure having opening at a distal end thereof. For example, the opening has a diameter of 1 millimeter or less, in another example the opening is 0.5 mm or less, in a further example 0.2 mm or less. The method includes placing the distal end of the flexible tapered polymer structure into contact with the tears adjacent the eye and applying negative pressure to the flexible tapered polymer structure. The method further includes buffering the sample of tears by addition of a buffering solution followed by analyzing the sample of tears to identify cannabinoids within the sample of tears and presenting results of analysis of the sample of tears.

The above summary is not intended to describe each illustrated embodiment or every implementation of the subject matter hereof. The figures and the detailed description that follow more particularly exemplify various embodiments.

### BRIEF DESCRIPTION OF THE DRAWINGS

Subject matter hereof may be more completely understood in consideration of the following detailed description of various embodiments in connection with the accompanying figures, in which:
FIG. 1 is a schematic depiction of the eye and tear film including the tear film layers;
FIG. 2 is a block diagram of a tear film sampling and a testing device which can be used to implement the method according to an example embodiment of the invention;
FIG. 3 is a flow chart depicting a sampling and testing method according to an example embodiment of the invention;
FIG. 4 is a flow chart depicting a sampling and testing method according to an example embodiment of the invention; and
FIG. 5 is a depiction of a sampling device which can be used to implement the method according to an example embodiment of the invention.

While various embodiments are amenable to various modifications and alternative forms, specifics thereof have been shown by way of example in the drawings and will be described in detail. It should be understood, however, that the intention is not to limit the claimed inventions to the particular embodiments described.

### DETAILED DESCRIPTION

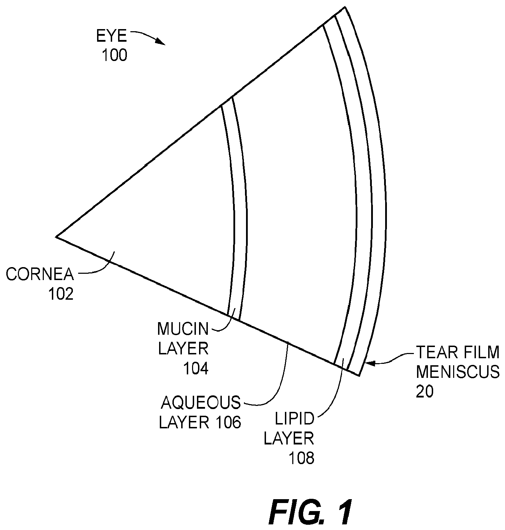

Referring to FIG. 1, the tear film is schematically depicted within an eye 100. The tear film generally is considered to include 3 layers. Starting from the surface of the cornea 102 these layers include the mucin layer 104, the aqueous layer 106, and a lipid layer 108. Because of gravity, some of the tear film tends to accumulate at the junction where the cornea meets the lower eyelid in what is commonly referred to as the tear meniscus 20.

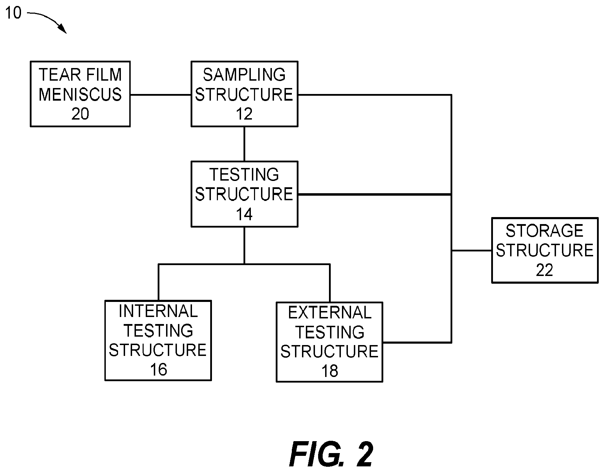

Referring to FIG. 2, a block diagram of tear film cannabis testing device 10 is depicted. Testing device 10 generally includes sampling structure 12 and testing structure 14. Testing structure 14 may include internal testing structure 16, external testing structure 18, or both. Sampling structure 12 is utilized to obtain a sample from the tear film meniscus 20. Testing device 10 may also include a storage structure 22 configured to store at least one of the sampling structure 12 and the testing structure 14. For example, storage structure 22 may be configured to store external testing structure 18.

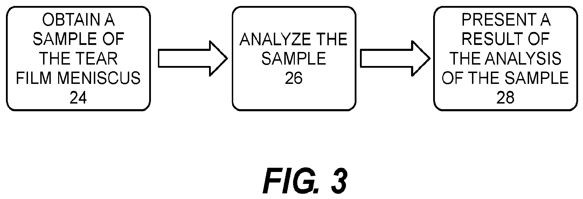

Referring to FIG. 3, a flowchart of a method according to an example embodiment of the invention is depicted. A sample of the tear film meniscus 20 is obtained (step 24) via the sampling structure 12 and analyzed (step 26) via the testing structure 14. In some embodiments, the sample is analyzed using internal testing structure 14. In other embodiments, the sample is analyzed using external testing structure 16. Embodiments of the method may also include presenting results of the analysis at step 28.

According to example embodiments of the invention, tear samples may be obtained by use of a micropipette or a capillary tube. The micropipette or capillary tube should be packaged in a sterile fashion for single use.

According to an example embodiment of the invention, the presence of cannabinoids in the tear film can be analyzed by application of color testing, gas chromatography, high-performance liquid chromatography or high-pressure liquid chromatography, gas chromatography-flame ionization detection ("GC-FID"), thin layer chromatography ("TLC"), spectrometry, or mass spectrometry.

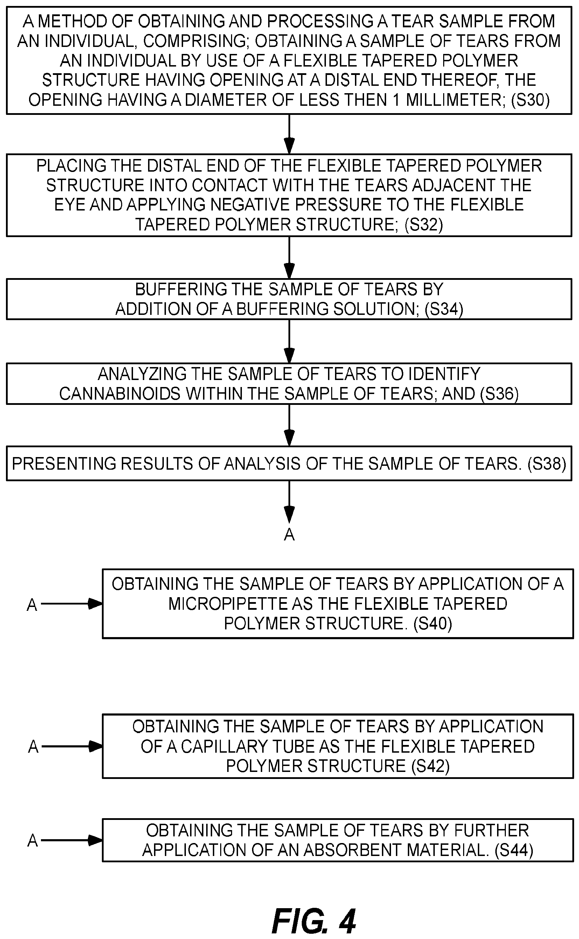

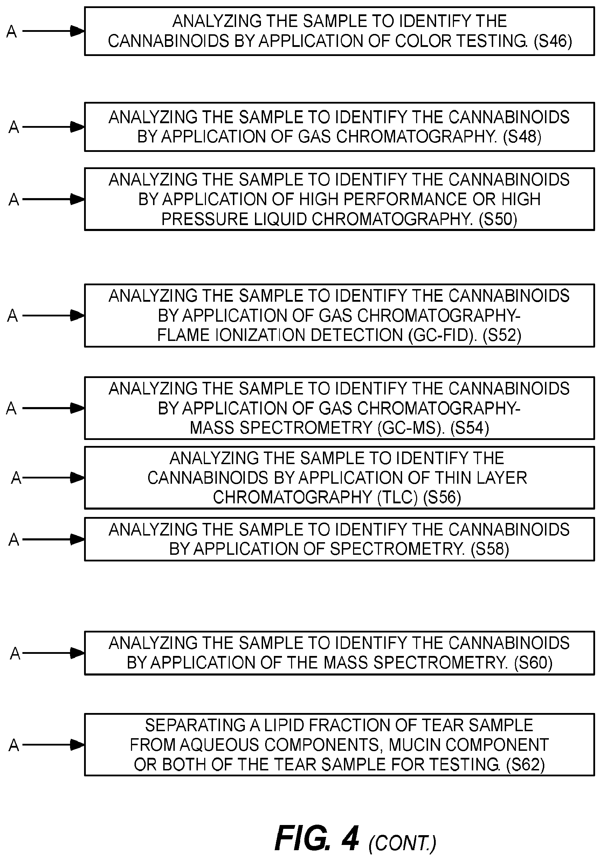

Referring to FIG. 4, according to another example embodiment of the invention, a method of obtaining and processing a tear sample from an individual, includes obtaining a sample of tears from an individual by use of a flexible tapered polymer structure having opening at a distal end thereof. S30 The opening has a diameter of 1 millimeter or less, in another example the opening is 0.5 mm or less, in a further example 0.2 mm or less. The method includes placing the distal end of the flexible tapered polymer structure into contact with the tears adjacent the eye and applying negative pressure to the flexible tapered polymer structure. S32 The method further includes buffering the sample of tears by addition of a buffering solution S34 followed by analyzing the sample of tears to identify cannabinoids within the sample of tears S36 and presenting results of analysis of the sample of tears S38.

The method may further include obtaining the sample of tears by application of a micropipette as the flexible tapered polymer structure. S40

The method may also include obtaining the sample of tears by application of a capillary tube as the flexible tapered polymer structure. S42

A not claimed method may include obtaining the sample of tears by further application of an absorbent material. S44

In another embodiment, the method may further include analyzing the sample to identify the cannabinoids by application of color testing. S46

In other example embodiments the method may include analyzing the sample to identify the cannabinoids by application of gas chromatography S48; by application of high performance or high pressure liquid chromatography S50; by application of gas chromatography-flame ionization detection (GC-FID) S52; by application of gas chromatography-mass spectrometry (GC-MS) S54; or by application of thin layer chromatography (TLC) S56.

Example methods may include analyzing the sample to identify the cannabinoids by application of spectrometry S58; or by application of the mass spectrometry S60.

The method may include separating a lipid fraction of the tear sample from aqueous components, mucin component or both of the tear sample for testing. S62.

A device that facilitates detecting cannabinoids in tears may include a sampling structure including a flexible tapered polymer structure having opening at a distal end thereof, the opening having a diameter of less than 1 millimeter; a sampling device applying negative pressure to the flexible tapered polymer structure and a container adapted to store a buffering solution formulated to buffer the sample of tears for testing. The sampling structure may be internal to the device. The device may include the storage structure being configured to receive the sampling structure and the testing structure. The sampling structure may be external.

The invention deals with a method of detecting cannabinoids including obtaining a sample of tears from an individual; and analyzing the sample of tears to identify cannabinoids within the sample of tears.

In yet a further example embodiment of the invention the method of identifying cannabinoids in tear film may further comprise separating a lipid fraction of the tear sample from an aqueous component, a mucin component or both the aqueous and mucin component of the tear sample prior to analysis for the presence of cannabinoids. In this exemplary embodiment, the storage structure 22 may be configured to store the materials required to perform the separation and store the resultant components.

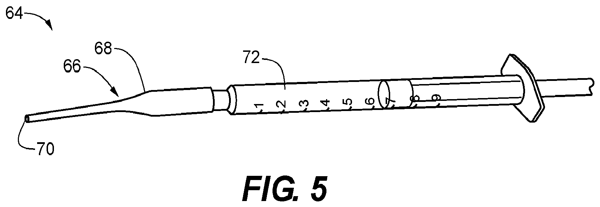

Referring to FIG. 5, a device 64 that facilitates detecting cannabinoids in tears includes a sampling structure 66 including a flexible tapered polymer structure 68 having opening 70 at a distal end thereof, opening 70 having a diameter of less than 1 millimeter; a sampling device 72 structured to apply negative pressure to the flexible tapered polymer structure 68; a container adapted to store a buffering solution formulated to buffer the sample of tears for testing; and a structure that is adapted for analyzing the sample of tears to identify cannabinoids within the sample of tears; and to present results of analysis of the sample of tears.

For example, the sampling structure may be internal to the device.

According to an example, a storage structure may be configured to receive the sampling structure and the testing structure.

Various embodiments of systems, devices, and methods have been described herein. These embodiments are given only by way of example and are not intended to limit the scope of the claimed invention.

It should be appreciated, moreover, that the various features of the embodiments that have been described may be combined in various ways to produce numerous additional embodiments. Moreover, while various materials, dimensions, shapes, configurations and locations, etc. have been described for use with disclosed embodiments, others besides those disclosed may be utilized without exceeding the scope of the claimed invention which is exclusively defined by the claims.

Persons of ordinary skill in the relevant arts will recognize that the subject matter hereof may comprise fewer features than illustrated in any individual embodiment described above. The embodiments described herein are not meant to be an exhaustive presentation of the ways in which the various features of the subject matter hereof may be combined. Accordingly, the embodiments are not mutually exclusive combinations of features; rather, the various embodiments can comprise a combination of different individual features selected from different individual embodiments, as understood by persons of ordinary skill in the art. Moreover, elements described with respect to one embodiment can be implemented in other embodiments even when not described in such embodiments unless otherwise noted.

## Claims

1. A method of obtaining and processing a tear sample from an individual, comprising
obtaining a sample of tears from a tear film meniscus (20) of an individual by use of a flexible tapered polymer structure (68) having opening (70) at a distal end thereof, the opening (70) having a diameter of 1 millimeter or less;
placing the distal end of the flexible tapered polymer structure (68) into contact with the tears adjacent the eye (100) and applying negative pressure to the flexible tapered polymer structure (68);
buffering the sample of tears by addition of a buffering solution (S34);
analyzing the sample of tears to identify cannabinoids within the sample of tears; and
presenting results of analysis of the sample of tears.

2. The method as claimed in claim 1, further comprising obtaining the sample of tears by application of a micropipette as the flexible tapered polymer structure (68).

3. The method as claimed in claim 1, further comprising obtaining the sample of tears by application of a capillary tube as the flexible tapered polymer structure (68).

4. The method as claimed in claim 1, further comprising obtaining the sample of tears by further application of an absorbent material.

5. The method as claimed in claim 1, further comprising analyzing the sample to identify the cannabinoids by application of color testing.

6. The method as claimed in claim 1, further comprising analyzing the sample to identify the cannabinoids by application of gas chromatography.

7. The method as claimed in claim 1, further comprising analyzing the sample to identify the cannabinoids by application of high performance or high pressure liquid chromatography.

8. The method as claimed in claim 1, further comprising analyzing the sample to identify the cannabinoids by application of gas chromatography-flame ionization detection (GC-FID).

9. The method as claimed in claim 1, further comprising analyzing the sample to identify the cannabinoids by application of gas chromatography-mass spectrometry (GC-MS).

10. The method as claimed in claim 1, further comprising analyzing the sample to identify the cannabinoids by application of thin layer chromatography (TLC).

11. The method as claimed in claim 1, further comprising analyzing the sample to identify the cannabinoids by application of spectrometry.

12. The method as claimed in claim 1, further comprising analyzing the sample to identify the cannabinoids by application of the mass spectrometry.

13. The Method as claimed in claim 1, further comprising separating a lipid fraction of the tear sample from aqueous components, mucin component or both of the tear sample prior to the analyzing step.

14. The Method as claimed in claim 1, wherein the opening (70) has a diameter of 0.5 mm or less.

15. The Method as claimed in claim 14, wherein the opening (70) has a diameter of 0.2 mm or less.

## Patentansprüche

1. Verfahren zum Gewinnen und Verarbeiten einer Tränenprobe von einer Person, umfassend Gewinnen einer Tränenprobe aus einem Tränenfilmmeniskus (20) einer Person unter Verwendung einer flexiblen, sich verjüngenden Polymerstruktur (68) mit einer Öffnung (70) an ihrem distalen Ende, wobei die Öffnung (70) einen Durchmesser von 1 Millimeter oder weniger aufweist;
Bringen des distalen Ende der flexiblen, sich verjüngenden Polymerstruktur (68) in Kontakt mit den Tränen neben dem Auge (100) und Ausüben von Unterdruck auf die flexible, sich verjüngende Polymerstruktur (68);
Puffern der Tränenprobe durch Zugabe einer Pufferlösung (S34);
Analysieren der Tränenprobe, um Cannabinoide in der Tränenprobe zu identifizieren; und
Präsentieren der Ergebnisse der Analyse der Tränenprobe.

2. Verfahren nach Anspruch 1, das ferner das Gewinnen der Tränenprobe durch Anwendung einer Mikropipette als flexible, sich verjüngende Polymerstruktur (68) umfasst.

3. Verfahren nach Anspruch 1, das ferner das Gewinnen der Tränenprobe durch Anwendung eines Kapillarrohrs als flexible, sich verjüngende Polymerstruktur (68) umfasst.

4. Verfahren nach Anspruch 1, das ferner das Gewinnen der Tränenprobe durch weitere Anwendung eines absorbierenden Materials umfasst.

5. Verfahren nach Anspruch 1, das ferner das Analysieren der Probe zum Identifizieren der Cannabinoide durch Anwendung eines Farbtests umfasst.

6. Verfahren nach Anspruch 1, das ferner das Analysieren der Probe zum Identifizieren der Cannabinoide durch Anwendung von Gaschromatographie umfasst.

7. Verfahren nach Anspruch 1, das ferner das Analysieren der Probe zum Identifizieren der Cannabinoide durch Anwendung von Hochleistungs- oder Hochdruckflüssigkeitschromatographie umfasst.

8. Verfahren nach Anspruch 1, das ferner das Analysieren der Probe zum Identifizieren der Cannabinoide durch Anwendung von Gaschromatographie-Flammenionisationsdetektion (GC-FID) umfasst.

9. Verfahren nach Anspruch 1, das ferner das Analysieren der Probe zum Identifizieren der Cannabinoide durch Anwendung von Gaschromatographie-Massenspektrometrie (GC-MS) umfasst.

10. Verfahren nach Anspruch 1, das ferner das Analysieren der Probe zum Identifizieren der Cannabinoide durch Anwendung von Dünnschichtchromatographie (TLC) umfasst.

11. Verfahren nach Anspruch 1, das ferner das Analysieren der Probe zum Identifizieren der Cannabinoide durch Anwendung von Spektrometrie umfasst.

12. Verfahren nach Anspruch 1, das ferner das Analysieren der Probe zum Identifizieren der Cannabinoide durch Anwendung der Massenspektrometrie umfasst.

13. Verfahren nach Anspruch 1, das ferner das Trennen einer Lipidfraktion der Tränenprobe von wässrigen Komponenten, der Muzinkomponente oder beiden Komponenten der Tränenprobe vor dem Analyseschritt umfasst.

14. Verfahren nach Anspruch 1, wobei die Öffnung (70) einen Durchmesser von 0,5 mm oder weniger aufweist.

15. Verfahren nach Anspruch 14, wobei die Öffnung (70) einen Durchmesser von 0,2 mm oder weniger aufweist.

## Revendications

1. Procédé d'obtention et de traitement d'un échantillon de larmes provenant d'un individu, comprenant
l'obtention d'un échantillon de larmes à partir d'un ménisque du film lacrymal (20) d'un individu à l'aide d'une structure polymère effilée flexible (68) comportant une ouverture (70) à son extrémité distale, l'ouverture (70) ayant un diamètre de 1 millimètre ou moins;
la mise en contact de l'extrémité distale de la structure polymère flexible effilée (68) avec les larmes adjacentes à l'œil (100) et l'application d'une pression négative à la structure polymère flexible effilée (68);
tamponner l'échantillon de larmes par ajout d'une solution tampon (S34);
analyser l'échantillon de larmes pour identifier les cannabinoïdes dans l'échantillon de larmes ; et
présenter les résultats de l'analyse de l'échantillon de larmes.

2. Procédé selon la revendication 1, comprenant en outre l'obtention de l'échantillon de larmes par application d'une micropipette en tant que structure polymère effilée flexible (68).

3. Procédé selon la revendication 1, comprenant en outre l'obtention de l'échantillon de larmes par application d'un tube capillaire en tant que structure polymère effilée flexible (68).

4. Procédé selon la revendication 1, comprenant en outre l'obtention de l'échantillon de larmes par application supplémentaire d'un matériau absorbant.

5. Procédé selon la revendication 1, comprenant en outre l'analyse de l'échantillon pour identifier les cannabinoïdes par application d'un test colorimétrique.

6. Procédé selon la revendication 1, comprenant en outre l'analyse de l'échantillon pour identifier les cannabinoïdes par application de la chromatographie en phase gazeuse.

7. Procédé selon la revendication 1, comprenant en outre l'analyse de l'échantillon pour identifier les cannabinoïdes par application de la chromatographie liquide haute performance ou haute pression.

8. Procédé selon la revendication 1, comprenant en outre l'analyse de l'échantillon pour identifier les cannabinoïdes par application d'une chromatographie en phase gazeuse avec détection par ionisation de flamme (GC-FID).

9. Procédé selon la revendication 1, comprenant en outre l'analyse de l'échantillon pour identifier les cannabinoïdes par application d'une chromatographie en phase gazeuse avec spectrométrie de masse (GC-MS).

10. Procédé selon la revendication 1, comprenant en outre l'analyse de l'échantillon pour identifier les cannabinoïdes par application d'une chromatographie sur couche mince (CCM).

11. Procédé selon la revendication 1, comprenant en outre l'analyse de l'échantillon pour identifier les cannabinoïdes par application d'une spectrométrie.

12. Procédé selon la revendication 1, comprenant en outre l'analyse de l'échantillon pour identifier les cannabinoïdes par application de la spectrométrie de masse.

13. Procédé selon la revendication 1, comprenant en outre la séparation d'une fraction lipidique de l'échantillon de larmes des composants aqueux, du composant mucine ou des deux composants de l'échantillon de larmes avant l'étape d'analyse.

14. Procédé selon la revendication 1, dans lequel l'ouverture (70) a un diamètre de 0,5 mm ou moins.

15. Procédé selon la revendication 14, dans lequel l'ouverture (70) a un diamètre de 0,2 mm ou moins.
